(19) Europäisches Patentamt
European Patent Office
Office européen des brevets

(11) **EP 1 463 939 B1**

(12) **EUROPÄISCHE PATENTSCHRIFT**

(45) Veröffentlichungstag und Bekanntmachung des Hinweises auf die Patenterteilung:
**26.03.2008 Patentblatt 2008/13**

(21) Anmeldenummer: **02792678.1**

(22) Anmeldetag: **19.12.2002**

(51) Int Cl.:
**G01N 33/18** (2006.01)   **G01N 33/02** (2006.01)
**G01N 27/62** (2006.01)

(86) Internationale Anmeldenummer:
**PCT/DE2002/004646**

(87) Internationale Veröffentlichungsnummer:
**WO 2003/058234 (17.07.2003 Gazette 2003/29)**

(54) **VERFAHREN ZUR IDENTIFIZIERUNG VON STOFFEN, DIE MIT DEUTERIERTEM WASSER MARKIERT, UND DEREN WASSERSTOFF- UND SAUERSTOFFISOTOPENVERHÄLTNISSE MASSENSPEKTROSKOPISCH BESTIMMT WERDEN**

METHOD FOR IDENTIFYING SUBSTANCES, WHICH ARE MARKED WITH DEUTERATED WATER AND WHOSE HYDROGEN AND OXYGEN ISOTOPE RATIOS ARE DETERMINED BY MEANS OF MASS SPECTROSCOPY

PROCEDE POUR IDENTIFIER DES SUBSTANCES MARQUEES AVEC DE L'EAU DEUTERISEE ET DONT LES RAPPORTS ENTRE L'ISOTOPE D'HYDROGENE ET L'ISOTOPE D'OXYGENE SONT DETERMINES PAR SPECTROSCOPIE DE MASSE

(84) Benannte Vertragsstaaten:
**AT BE BG CH CY CZ DE DK EE ES FI FR GB GR IE IT LI LU MC NL PT SE SI SK TR**

(30) Priorität: **11.01.2002 DE 10200802**

(43) Veröffentlichungstag der Anmeldung:
**06.10.2004 Patentblatt 2004/41**

(73) Patentinhaber: **Forschungszentrum Jülich GmbH 52425 Jülich (DE)**

(72) Erfinder: **BONER, Markus 52428 JÜLICH (DE)**

(56) Entgegenhaltungen:
**WO-A-02/50560          US-A- 5 979 228**

- **SCHOELLER D A ET AL: "TOTAL BODY WATER MEASUREMENT IN HUMANS WITH OXYGEN-18 LABELED AND DEUTERIUM LABELED WATER" AMERICAN JOURNAL OF CLINICAL NUTRITION, Bd. 33, Nr. 12, 1980, Seiten 2686-2693, XP009009038 ISSN: 0002-9165**
- **ROSSMANN A ET AL: "MULTIELEMENT STABLE ISOTOPE RATIO ANALYSIS OF GLYCEROL TO DETERMINEITS ORIGIN IN WINE" ZEITSCHRIFT FUER LEBENSMITTEL-UNTERSUCHUNG UND -FORSCHUNG. A, EUROPEAN FOOD RESEARCH AND TECHNOLOGY, SPRINGER, HEIDELBERG, DE, Bd. 207, Nr. 3, 1998, Seiten 237-243, XP000982669 ISSN: 1431-4630**
- **KOZIET J ET AL: "Determination of the oxygen-18 and deuterium content of fruit and vegetable juice water. An European inter-laboratory comparison study" ANALYTICA CHIMICA ACTA 1995 NETHERLANDS, Bd. 302, Nr. 1, 1995, Seiten 29-37, XP001146618 ISSN: 0003-2670**
- **PUPIN A M ET AL: "Use of isotopic analyses to determine the authenticity of Brazilian orange juice (Citrus sinensis)." JOURNAL OF AGRICULTURAL AND FOOD CHEMISTRY, Bd. 46, Nr. 4, April 1998 (1998-04), Seiten 1369-1373, XP001146617 ISSN: 0021-8561**
- **ROSSMANN A ET AL: "Stable oxygen isotope content of water of EU data-bank wines from Italy, France and Germany" ZEITSCHRIFT FUER LEBENSMITTEL-UNTERSUCHUNG UND -FORSCHUNG. A, EUROPEAN FOOD RESEARCH AND TECHNOLOGY, SPRINGER, HEIDELBERG, DE, Bd. 208, Nr. 5-6, 1999, Seiten 400-407, XP002215533 ISSN: 1431-4630**

• DUNBAR J ET AL: "OXYGEN AND HYDROGEN ISOTOPES IN FRUIT AND VEGETABLES JUICES" PLANT PHYSIOLOGY (BETHESDA), Bd. 72, Nr. 3, 1983, Seiten 725-727, XP001147341 ISSN: 0032-0889

**Beschreibung**

Die Erfindung betrifft ein Verfahren zur Identifizierung von Stoffen, die mit deuteriertem Wasser markiert sind.

Bei Lebensmitteln, Futtermitteln und Arzneimitteln besteht der Bedarf, einen eindeutigen Nachweis über die Herkunft zu haben. So ist es beispielsweise für Reklamationszwecke erforderlich nachzuweisen, von welchem Lieferanten ein Rohstoff geliefert wurde. Umgekehrt wünschen sich Lieferanten die Möglichkeit durch eindeutigen Herkunftsnachweis um sich vor Schadensansprüchen zu schützen, die fälschlicherweise an sie gerichtet wurden. Nach dem Stand der Technik ist es bekannt, die gelieferten Stoffe durch Markierung mit Isotopen zu kennzeichnen. Hierzu werden Substanzen eingesetzt, welche mit den schweren stabilen Isotopen der Bioelemente $^{13}C$, $^{15}N$, $^{16}O$ oder $^2H$ angereichert sind. Diese stabilen Isotope kommen in der Natur und damit auch in den zu markierenden Stoffen in natürlichen Verteilungsbreiten vor, so daß sehr hohe Konzentrationen der markierten Substanzen zugesetzt werden müssen um eine signifikante Abweichung zu bewirken. Bei der Markierung nach dem Stand der Technik muß auch der Problematik Rechnung getragen werden, daß die Proben, deren Herkunft identifiziert werden soll gegebenenfalls auch verdünnt werden. Dies ist beispielsweise bei Fruchtsäften denkbar. Auch nach der Verdünnung muß die Konzentration von stabilem Isotop immer noch signifikant über der natürlich vorkommenden Konzentration liegen, damit ein Nachweis über die Herkunft erfolgen kann. Die Folge ist, daß entsprechend hohe Konzentrationen markierter Substanz eingesetzt werden müssen. Dies hat jedoch einen hohen Verbrauch an markierter Substanz und damit verbunden hohe Kosten zur Folge. Weiterhin können insbesondere bei Nahrungsmitteln durch unerwünscht hohe Konzentrationen an markierter Substanz toxische Nebenwirkungen auftreten, die nicht akzeptabel sind. Ist die Verdünnung beliebig hoch, so kann die Markierung gegebenenfalls nicht mehr gemessen werden.

WO0250560 offenbart ein Verfahren zur Ermittlung der Herkunft biologisch, biochemisch und ernährungsphysiologisch relevanter Stoffe und deren Unterscheidung von synthetischen Analoga unter Ausnutzung ihrer relativen positionellen Sauerstoff- bzw. Wasserstoff-Isotopenverhältnisse, mit denen man die Authentizität bzw. Herkunft oder Fälschung des Stoffes erkennen kann.

Rossman (Rossman A. et al; T. Lebensm. Unters. Forsch. A (1999) 208:400-407) beschreibt eine Methode zur Bestimmung der Authentizizät von europäischen Weinen durch die Bestimmung der relativen Sauerstoff- bzw. Wasserstoff-Isotopenverhältnisse.

Es ist daher die Aufgabe der Erfindung ein universelles Verfahren zur Identifizierung von Substanzen zu schaffen, welches kostengünstig ist und bei dem keine toxischen Stoffe eingesetzt werden müssen. Weiterhin soll das Verfahren auch eine Identifizierung ermöglichen, wenn die mit der Markersubstanz versehene Substanz extrem verdünnt worden ist, so daß sich das Vorkommen des stabilen Isotops in der Probe nicht mehr von der natürlichen Konzentration des stabilen Isotops in der Natur unterscheidet.

Ausgehend vom Oberbegriff des Anspruchs 1 wird die Aufgabe, mit den im kennzeichnenden Teil des Anspruchs 1 angegebenen Merkmalen, erfindungsgemäß gelöst.

Mit dem erfindungsgemäßen Verfahren ist es nunmehr möglich, die Herkunft von Stoffen kostengünstig und sehr genau zu bestimmen. Toxische Nebenwirkungen werden vermieden. Weiterhin wird die Zuverlässigkeit der Methode selbst bei extremer Verdünnung der Probe nicht gemindert.

Vorteilhafte Weiterbildungen des erfindungsgemäßen Verfahrens sind in den Unteransprüchen angegeben.

Im Folgenden soll die Erfindung beschrieben werden.

Die Figuren zeigen verschiedene Graphen.

Es zeigt:

Fig.1: Abhängigkeit der $^{18}O$-Konzentration von der D-Konzentration in natürlichem Wasser.

Fig.2: Einfluß der Verdünnung auf die Markierung nach dem Stand der Technik am Beispiel von Fruchtsaft.

Fig.3: Minimale und maximale Bereiche für δ-Werte für natürliche Proben von Wasser, weltweit und regional (Deutschland).

Tabelle 1: δ-Werte für verschiedene internationale Standardwasser.

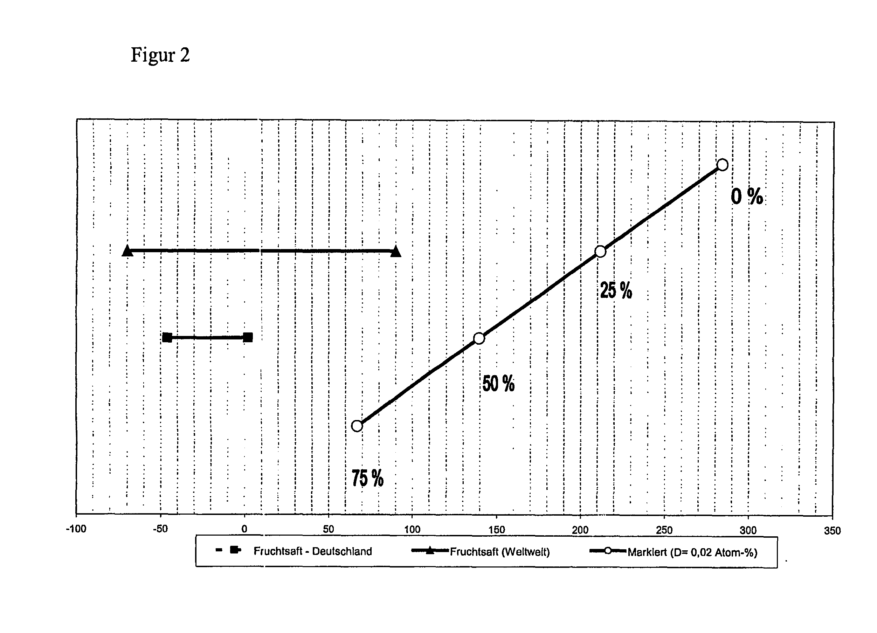

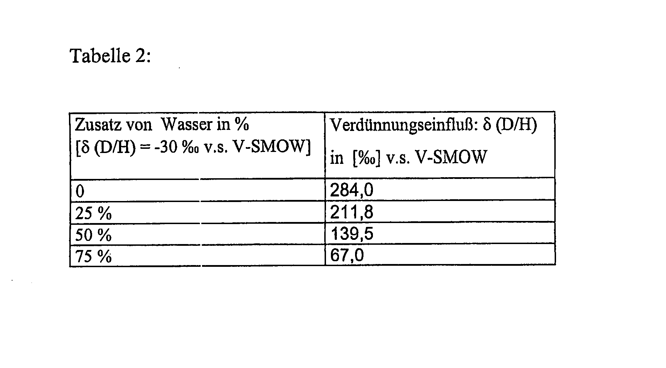

Tabelle 2: Verdünnungsreihe gemäß Figur 2.

Tabelle 3: Prozentuale Häufigkeit von D in Wasser regionaler Herkunft (DE).

Die Isotopenverhältnisse von H/D und $^{18}O/^{16}O$ für Wasser korrelieren in der Natur. Das heißt in natürlich vorkommendem Wasser ist einer erhöhten Konzentration an D eine höhere Konzentration an $O^{18}$ zugeordnet. Der Zusammenhang wir durch die Gesetzmäßigkeit nach Formel 1 beschrieben.

$$\delta D = 8 * \delta^{18}O + 10$$

Formel 1

Die in Formel 1 beschriebene Funktion ist eine Gerade und wird als "Meteoric Water Line" bezeichnet. Auf ihr befinden sich die in der Natur vorkommenden Zusammensetzungen von Wasser bezüglich des Verhältnisses H/D /$^{18}O/^{16}O$.

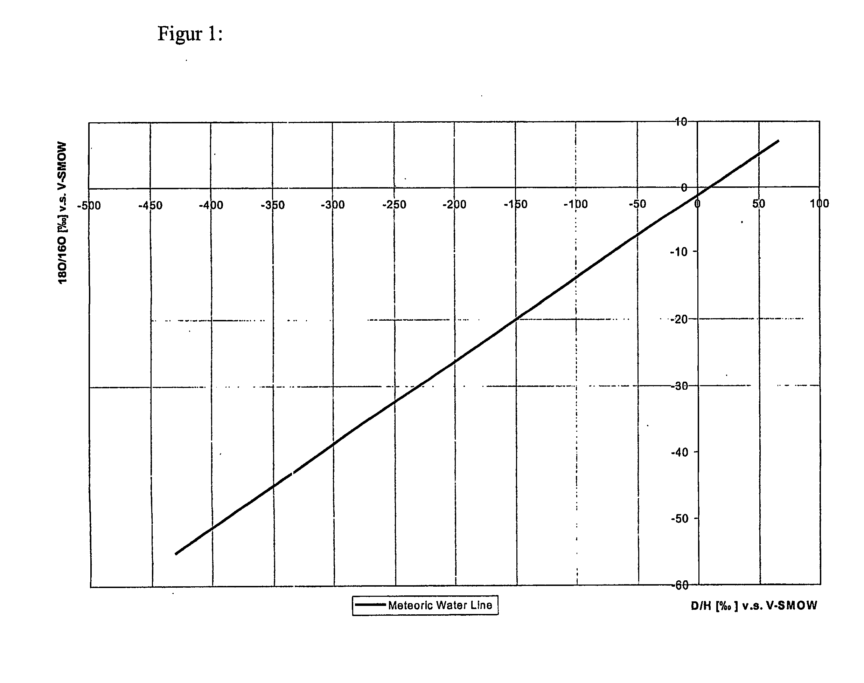

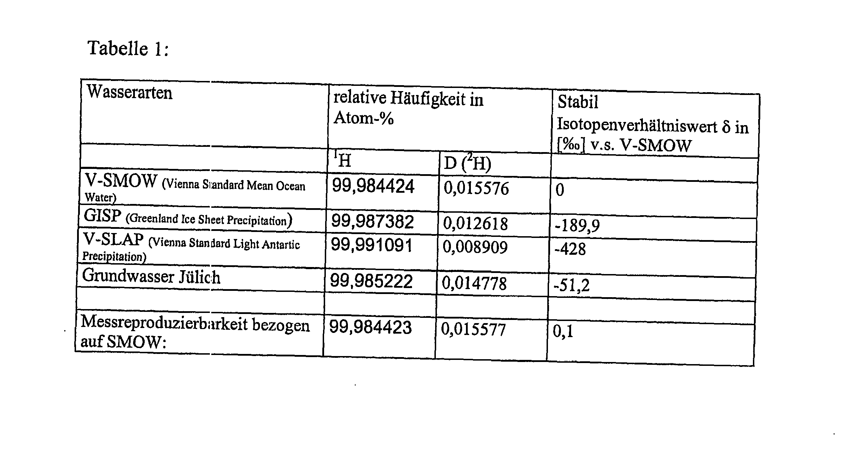

Der Graph ist in Figur 1 dargestellt. In ihm ist die Abszisse die Konzentration $^{18}O/^{16}O$ in Promille und die Ordinate die Konzentration H/D in Promille. In Tabelle 1 sind Beispiele für verschiedene Sorten von Wasser gezeigt. In der Spalte "Verbindung" befinden sich die verschiedenen Wasserproben SMOW, ein Wasserstandard, dessen Verhältnis D/H per Definition auf null gesetzt wird. Darunter sind Abweichungen von SMOW in Promille sowie die Reproduzierbarkeit der massenspektrometrischen Messungen der Verhältnisse D/H angegeben. In Spalte 1 sind weiterhin GISP = Grönlandwasser, Slap = Antarktiswasser und zum Vergleich Wasser aus Jülich. Spalte 2 gibt die relative Häufigkeit von H und D in Atom-% an, und in Spalte 3 ist das Verhältnis $\delta$ in Promille. Das Verhältnis $\delta$ aus Formel 1 wird massenspektroskopisch gemäß Formel 2 bestimmt. Notwendig ist hier für die exakte Bestimmung der stabilen Isotopenverhältnisse von H/D und $^{18}O/^{16}O$. Hierzu werden vorzugsweise Stabil Isotopen Massenspektrometer (IRMS) eingesetzt.

$$\delta = \frac{R_{Probe} - R_{Standard}}{R_{Standard}} \times 1000 \quad \text{in Promille}$$

$$\text{Mit } R = D/H \; ; \quad \text{oder } ^{18}O/^{16}O$$

Formel 2

Erfindungsgemäß werden Proben, die identifizierbar gemacht werden sollen mit D angereichertem HDO oder $D_2O$ oder einem Gemisch aus HDO und $D_2O$ versetzt, deren 8-Wert bekannt ist. Die Proben können Lebensmittel, Arzneimittel oder Futtermittel sein. Wird von der mit angereichertem Wasser Probe ein Stabil Isotopen Massenspektrum aufgenommen, so weicht das Verhältnis H/D zu $^{18}O/^{16}O$ von den Werten auf der Meteoric Water Line ab und es ist der Nachweis erbracht, aus welcher Quelle die Probe bezogen wurde. Dieser Nachweis ist unabhängig davon, ob die Probe verdünnt wurde oder nicht, da das Verhältnis von H/D zu $^{18}O/^{16}O$ d.h. $\delta$ immer gleich bleibt. Bei einer Verdünnung mit natürlichem Wasser ändern sich zwar die Konzentrationen von D und $^{18}O$, jedoch nicht das Verhältnis von H/D zu $^{18}O/^{16}O$. Dies ist auch der Grund dafür, daß das mit D angereichterte Wasser, das zu Identifikationszwecken zugegeben wird, nur in geringen Mengen eingesetzt werden muß. Während nach dem Stand der Technik so viel markierte Substanz oder markiertes Wasser zugegeben werden muß, daß die. D-Konzentration weit über der natürlichen Konzentration des D in Wasser liegt, um auch bei Verdünnung noch einen Nachweis führen zu können, kommt man mit dem erfindungsgemäßen Verfahren von vorne herein mit wenig markiertem Wasser aus. Mit zu 98,8% angereichertem Wasser sind Markierungen in der Relation 1 1 angereichertes Wasser in 800000 l natürlichem Wasser nachweisbar, da es nicht auf die absolute Menge von D sondern auf das Verhältnis H/D zu $^{18}O/^{16}O$ ankommt.

Zum Vergleich wird in Figur 2 der Einfluß der Markierung nach dem Verfahren nach dem Stand der Technik gezeigt. In Figur 2 von Fruchtsäften ist auch der Abszisse die Bandbreite der Werte $\delta$ gegenüber dem Standard angegeben. Der Ordinate kommt kein besonderer Wert zu, jedoch sind in verschiedenen Abständen parallel zur Abszisse die Bereiche für verschiedene Fruchtsäfte angegeben. Die Linie mit der Steigung gibt die Werte $\delta$ für Wasser an, welches mit D angereichert wurde, und das verschiedenen Verdünnungen unterworfen wurde. Entlang der Verdünnungsreihe wird erkenntlich, daß die Werte $\delta$ gemäß dem Verfahren nach dem Stand der Technik nach links wandert und immer niedrigere Werte annehmen und letztlich bei einer Verdünnung auf 25% in einen Bereich hinein rutschen, der nicht mehr von nicht markierten Proben zu unterscheiden ist. Die der Figur 2 zugeordneten D/H-Werte der Verdünnungsreihe sind in Tabelle 2 wiedergegeben.

Bei dem erfindungsgemäßen Verfahren würde der Meßpunkt $\delta$ auch bei einer Verdünnung der Probe in Figur

weiterhin außerhalb der "Meteroic Water Line" verbleiben und somit Identifizierbarkeit gewährleisten.

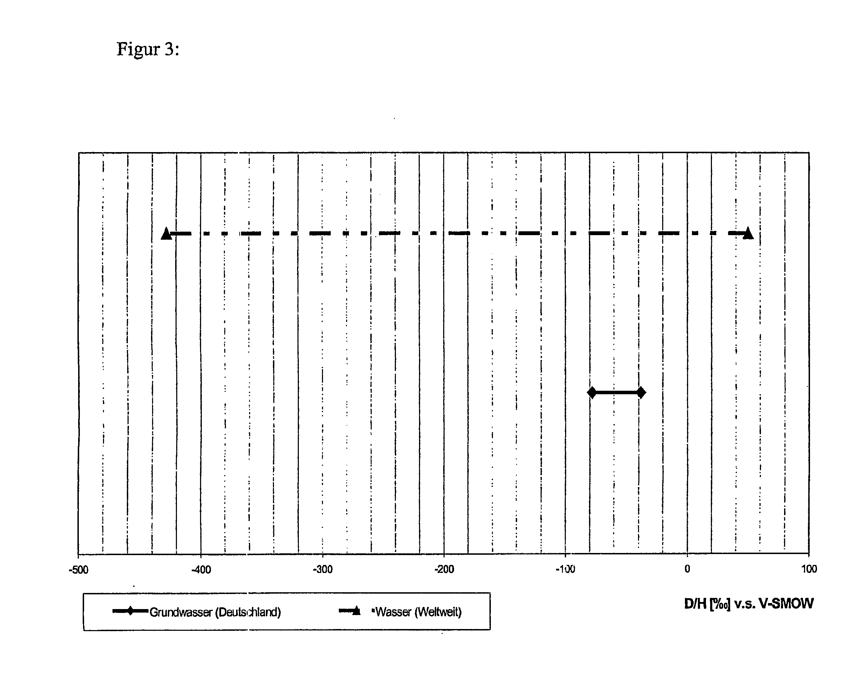

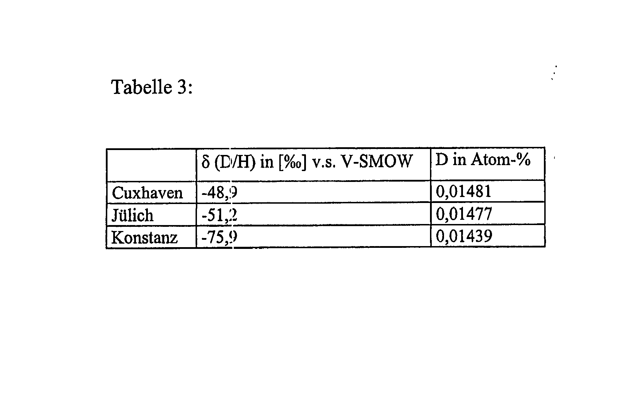

In Figur 3 sind die Bereiche der zu erwartenden Werte für δ für einige natürliche Proben angegeben. Die Abszisse ist wie in Figur 2 für δ-Werte gegen den Standard SMOW skaliert. Beispielhafte Wasserwerte aus dieser Figur sind in Tabelle 3 angegeben.

Das erfindungsgemäße Verfahren zum Nachweis der Herkunft von Stoffen mittels stabiler Isotope kann in der Futtermittel-, Lebensmittel-, und Arzneimittelidentifizierung auch in großtechnischem Maßstab eingesetzt werden. Es ist besonders kostengünstig, da nur sehr geringe Mengen mit Deuterium angereichertem Wasser eingesetzt werden müssen. Eine toxische Belastung der Proben findet nicht statt, da das markierte Wasser insbesondere in den geringen Mengen untoxisch ist. Die Methode ist unanfällig gegen Verdünnungseffekte. Verwenden zwei Hersteller markiertes Wasser verschiedenen Anreicherungsgrades, so kann die Herkunft unterschieden werden.

**Patentansprüche**

1. Verfahren zur Identifizierung von Stoffen, die mit deuteriertem Wasser markiert sind,
   **dadurch gekennzeichnet,**
   **daß** eine Probe des Stoffes, die mit Deuteriumhaltigem Wasser eines bekannten Deuteriumgehaltes versetzt ist einer massenspektroskopischen Untersuchung unterzogen wird, wobei der Gehalt an Deuterium (D), H und an $^{18}O$, $^{16}O$ bestimmt wird und
   durch Ermitteln des Verhältnisses von Deuterium und $^{18}O$ verglichen wird, ob das Verhältnis von D/H zu $^{18}O/^{16}O$ von dem natürlichen Verhältnis abweicht.

2. Verfahren nach Anspruch 1,
   **dadurch gekennzeichnet,**
   **daß** als Stoff eine Komponente aus der Gruppe Futtermittel, Lebensmittel und Arzneimittel eingesetzt wird.

3. Verfahren nach Anspruch 1 oder 2,
   **dadurch gekennzeichnet,**
   **daß** ein Stabil Isotop Massenspektrometer eingesetzt wird.

**Claims**

1. Method for identifying substances, which are marked with deuterated water
   **characterised in that**
   a sample of the substance, which is mixed with water containing deuterium with a known deuterium content, is subjected to a mass spectroscopic investigation, in which the deuterium (D), H and $^{18}O$, $^{16}O$ content is specified and by calculating the ratio of deuterium and $^{18}O$ it is compared whether the ratio of D/H to $^{18}O/^{16}O$ deviates from the natural ratio.

2. Method according to claim 1,
   **characterised in that**
   a component from the group of animal feedstuffs, foodstuffs and pharmaceutical products is used as the substance.

3. Method according to claim 1 or 2,
   **characterised in that**
   a stable isotope mass spectrometer is used.

**Revendications**

1. Procédé d'identification de substances qui sont marquées à l'eau deutériée,
   **caractérisé**
   **en ce que** l'on soumet un échantillon de la substance, qui est mélangé à de l'eau contenant du deutérium d'une teneur en deutérium connue, à une étude de spectroscopie de masse, la teneur en deutérium (D), H et en $^{18}O$, $^{16}O$ étant déterminée et, par détermination du rapport du deutérium et du $^{18}O$, on compare si le rapport de D/H au $^{18}O/^{16}O$ s'écarte du rapport naturel.

**2.** Procédé suivant la revendication 1,
**caractérisé**
**en ce qu'**on utilise comme substance un constituant du groupe aliments du bétail, denrées alimentaires et médicaments.

**3.** Procédé suivant la revendication 1 ou 2,
**caractérisé**
**en ce que** l'on utilise un spectromètre de masse Stabil Isotop.

Figur 1:

Tabelle 1:

| Wasserarten | relative Häufigkeit in Atom-% | | Stabil Isotopenverhältniswert δ in [‰] v.s. V-SMOW |
| --- | --- | --- | --- |
| | $^1$H | D ($^2$H) | |
| V-SMOW (Vienna Standard Mean Ocean Water) | 99,984424 | 0,015576 | 0 |
| GISP (Greenland Ice Sheet Precipitation) | 99,987382 | 0,012618 | -189,9 |
| V-SLAP (Vienna Standard Light Antartic Precipitation) | 99,991091 | 0,008909 | -428 |
| Grundwasser Jülich | 99,985222 | 0,014778 | -51,2 |
| | | | |
| Messreproduzierbarkeit bezogen auf SMOW: | 99,984423 | 0,015577 | 0,1 |

Figur 2

Tabelle 2:

| Zusatz von Wasser in % [$\delta$ (D/H) = -30 ‰ v.s. V-SMOW] | Verdünnungseinfluß: $\delta$ (D/H) in [‰] v.s. V-SMOW |
|---|---|
| 0 | 284,0 |
| 25 % | 211,8 |
| 50 % | 139,5 |
| 75 % | 67,0 |

Figur 3:

Tabelle 3:

| | δ (D/H) in [‰] v.s. V-SMOW | D in Atom-% |
|---|---|---|
| Cuxhaven | -48,9 | 0,01481 |
| Jülich | -51,2 | 0,01477 |
| Konstanz | -75,9 | 0,01439 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0250560 A

**In der Beschreibung aufgeführte Nicht-Patentliteratur**

- **ROSSMAN A. et al.** *T. Lebensm. Unters. Forsch.,* 1999, vol. 208, 400-407